# EUROPEAN PATENT SPECIFICATION

(11) **EP 3 927 340 B1**
(45) Date of publication and mention of the grant of the patent: **03.09.2025**
(21) Application number: 20759153.8
(22) Date of filing: 17.02.2020
(51) Int. Cl.: A61K 31/4184, A61P 1/04

(54) **PHARMACEUTICAL COMPOSITION COMPRISING TEGOPRAZAN FOR PREVENTING THE RECURRENCE OF GASTROESOPHAGEAL REFLUX DISEASE**
PHARMAZEUTISCHE ZUSAMMENSETZUNG MIT TEGOPRAZAN ZUR VERHINDERUNG DES WIEDERAUFTRETENS DER GASTROÖSOPHAGEALEN REFLUXKRANKHEIT
COMPOSITION PHARMACEUTIQUE COMPRENANT DU TEGOPRAZAN POUR PRÉVENIR LA RÉCIDIVE DU REFLUX GASTRO-OESOPHAGIEN

(30) Priority: 18.02.2019 KR 20190018801
(43) Date of publication of application: 29.12.2021
(73) Proprietor: HK inno.N Corporation, Seoul 04551 (KR)
(72) Inventor: KIM, Bong Tae, Hanam-si Gyeonggi-do 12904 (KR); LEE, Hyun Kyung, Gwangmyeong-si Gyeonggi-do 14230 (KR); NAM, Ji Yeon, Seoul 06298 (KR); OH, Mi Hwa, Seoul 05670 (KR); SONG, Geun Seog, Seoul 02834 (KR); KIM, Myeongjoong, Anyang-si Gyeonggi-do 14061 (KR)
(74) Representative: Kraus & Lederer PartGmbB
(86) International application number: PCT/IB2020/051296
(87) International publication number: WO 2020/170106

(56) References cited:
- EP-A1- 3 305 291
- WO-A1-2018/056720
- KR-A- 20180 075 235
- KR-B1- 101 088 247
- KR-B1- 101 829 706
- NOBUYUKI TAKAHASHI, YUKINORI TAKE: "Tegoprazan, a Novel Potassium-Competitive Acid Blocker to Control Gastric Acid Secretion and Motility", JOURNAL OF PHARMACOLOGY AND EXPERIMENTAL THERAPEUTICS, WILLIAMS AND WILKINS CO., vol. 364, no. 2, 1 February 2018 (2018-02-01), pages 275 - 286, XP055501129, ISSN: 0022-3565, DOI: 10.1124/jpet.117.244202

## Description

### [Technical Field]

The present invention relates to maintenance therapy for gastroesophageal reflux disease of a pharmaceutical composition comprising a benzimidazole derivative compound.

### [Background Art]

With the recent westernization of dietary life, gastroesophageal reflux disease has been on a rising trend in South Korea.

The gastroesophageal reflux disease is defined as symptoms or mucosal damage caused by the reflux of gastric acid into the esophagus, and its symptoms such as heartburn, acid regurgitation and the like become serious health problems, which lower the quality of life.

Out of contributing factors to causing gastroesophageal reflux, what matters is a transient lower esophageal sphincter relaxation. Accordingly, for treatment of gastroesophageal reflux disease, a method for inhibiting such transient lower esophageal sphincter relaxation may be theoretically taken into consideration. However, such method has yet to achieve a clinically large effect and has still remained in a research stage. Up to now, for the treatment of gastroesophageal reflux disease, the method for inhibiting a gastric acid secretion by using proton pump inhibitors, H2 receptor blockers, etc., has been used the most.

However, in case of patients with gastroesophageal reflux disease, almost 60 to 70% of them develop such gastroesophageal reflux disease again within a certain period of time, even after their symptoms are treated with administration of a gastric-acid secretion inhibitor for a certain period of time. Such a recurrence rate is similar not only in erosive gastroesophageal reflux disease but also in non-erosive gastroesophageal reflux disease.

As such, upon the recurrence of gastroesophageal reflux disease, even in case of administering the same dose of the gastric-acid secretion inhibitor as used to show an effect in a previous treatment, this administration may fail to fully exhibit the same effect as before, or symptoms thereof may get worse than before. In some cases, other complications may arise due to gastroesophageal reflux.

Thus, it may be also very important to safely and effectively prevent the recurrence of gastroesophageal reflux disease without any side effects, after such gastroesophageal reflux disease is treated.

Among the prior art, related applications for treatment of gastroesophageal reflux disease are disclosed in WO 2018/056720 A1, EP 3 305 291 A1, Takahashi et al.: J. Pharm. Exp. Ther. 2018, 364(2), 275-286, KR 101 829 706 B1, and KR 2018-0075235 A.

### [Prior Art Reference]

### [Patent Document]

Korea Patent Registration No. 10-1088247

### [Disclosure]

### [Technical Problem]

The present invention is to provide a pharmaceutical composition for use in a method of preventing the recurrence of gastroesophageal reflux disease, comprising tegoprazan which is a compound represented by the following chemical formula 1 or a pharmaceutically acceptable salt thereof in amount of 25mg as tegoprazan:

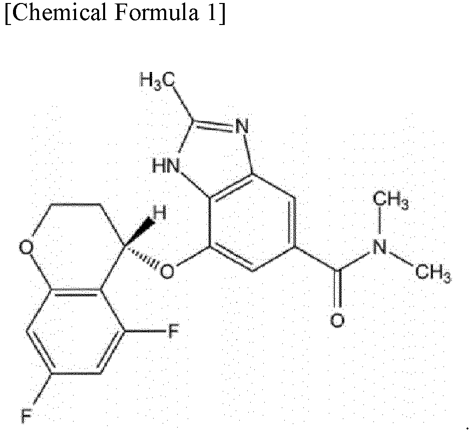

### [Technical Solution]

The present invention provides a pharmaceutical composition for use in a method of preventing the recurrence of gastroesophageal reflux disease, comprising tegoprazan which is a compound represented by the following chemical formula 1 or a pharmaceutically acceptable salt thereof in amount of 25mg as tegoprazan:

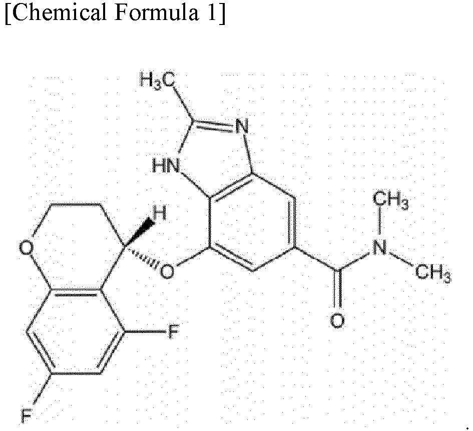

The pharmaceutical composition comprising tegoprazan or a pharmaceutically acceptable salt thereof in amount of 25mg as tegoprazan may effectively prevent the recurrence of gastroesophageal reflux disease.

The use of the pharmaceutical composition of the present invention comprising the tegoprazan or the pharmaceutically acceptable salt thereof in amount of 25mg as tegoprazan may prevent the recurrence of gastroesophageal reflux disease repeatedly. Also, the pharmaceutical composition of the present invention has excellent stability and thus may sufficiently prevent the recurrence of gastroesophageal reflux disease without any side effects, even in case of being taken for a long period of time.

The pharmaceutical composition of the present invention comprising the tegoprazan or the pharmaceutically acceptable salt thereof in amount of 25mg as tegoprazan is not affected by H. pylori infection or the polymorphism of CYP gene and may effectively and sufficiently prevent the recurrence of gastroesophageal reflux disease without any side effects.

In the present invention, tegoprazan which is a compound represented by the chemical formula 1 is also named "(S)-4-(5,7-difluorochroman-4-yloxy)-N,N,2-trimethyl-1H-benzo[d]imidazole-6-carboxamide)."

In the present invention, gastroesophageal reflux disease is a disease in which gastric acid or stomach contents rise up into the esophagus, resulting in a burning pain or soreness inside the chest, and may be non-erosive gastroesophageal reflux disease or erosive gastroesophageal reflux disease.

In the present invention, the term "a subject" may mean mammals including humans, and particularly may mean humans.

In the present invention, the pharmaceutical composition comprising 25 mg of tegoprazan may mean the pharmaceutical composition includes tegoprazan or a pharmaceutically acceptable salt thereof in such an amount as to be 25mg as tegoprazan.

In the present invention, whether gastroesophageal reflux disease is occurred or treated may be determined with endoscopy or depending on whether symptoms of gastroesophageal reflux disease is expressed or not. Particularly, erosive gastroesophageal reflux disease may be determined by the presence of erosion and by the presence of a symptom of heartburn and/or gastric acid reflux, and more particularly the occurrence and degree of erosive gastroesophageal reflux disease may be determined by whether or not it corresponds to A to D of LA grade through endoscopy.

In the present invention, preventing the recurrence of gastroesophageal reflux disease may include preventing the recurrence of gastroesophageal reflux disease, or inhibiting or delaying the recurrence of gastroesophageal reflux disease.

The present invention provides the pharmaceutical composition comprising 25 mg of tegoprazan, for use in a method of preventing the recurrence of gastroesophageal reflux disease, wherein the pharmaceutical composition is administered to a subject whose gastroesophageal reflux disease have been treated after being diagnosed with the gastroesophageal reflux disease.

In embodiments of the present invention, the subject to which the pharmaceutical composition comprising 25 mg of tegoprazan is administered may be one whose gastroesophageal reflux disease is identified as having been treated after the onset of it.

In the present invention, the subject may be one who is diagnosed with gastroesophageal reflux disease or experienced with symptoms of gastroesophageal reflux disease before administration of the pharmaceutical composition comprising 25 mg of tegoprazan, and particularly may be one who is diagnosed with non-erosive gastroesophageal reflux disease or erosive gastroesophageal reflux disease or experienced with a symptom such as heartburn/gastric acid reflux, etc.

In embodiments of the present invention, the subject may be one who is diagnosed with non-erosive gastroesophageal reflux disease before administration of the pharmaceutical composition comprising 25 mg of tegoprazan, and particularly may be one who has experienced symptoms such as heartburn/gastric acid reflux, though erosion is not observed by performing upper gastrointestinal endoscopy before administration of the pharmaceutical composition comprising 25 mg of tegoprazan.

In embodiments of the present invention, the subject may be one who is diagnosed with erosive gastroesophageal reflux disease before administration of the pharmaceutical composition comprising 25 mg of tegoprazan, and particularly may be one who is diagnosed with erosive gastroesophageal reflux disease corresponding to A to D of LA grade by performing upper gastrointestinal endoscopy before administration of the pharmaceutical composition comprising 25 mg of tegoprazan.

In embodiments of the present invention, the subject may be one who has experienced gastroesophageal reflux disease more than or equal to once or twice. For example, the subject may be one who has experienced the recurrence of gastroesophageal reflux disease.

In the present invention, a state in which the subject's gastroesophageal reflux disease has been treated may mean the state without symptoms of gastroesophageal reflux disease. Particularly, a state in which the subject's gastroesophageal reflux disease has been treated may mean the state without any more symptoms of gastroesophageal reflux disease such as heartburn and/or gastric acid reflux.

In embodiments of the present invention, a state in which the subject's gastroesophageal reflux disease has been treated may be identified by the presence of erosion through gastroscopy for the subject. Particularly, a state in which the subject's gastroesophageal reflux disease has been treated may mean the state in which erosion is not observed by performing upper gastrointestinal endoscopy for the subject. More particularly, a state in which the subject's gastroesophageal reflux disease has been treated may mean the state not corresponding to anywhere from A to D of LA grade by performing upper gastrointestinal endoscopy for the subject.

In the present invention, the recurrence of gastroesophageal reflux disease may mean the state in which, after the subject diagnosed with gastroesophageal reflux disease have been treated through drug treatment, etc., the subject is diagnosed with the onset of gastroesophageal reflux disease again or occurred symptoms of gastroesophageal reflux disease such as heartburn or gastric acid reflux again.

In the present invention, the recurrence of gastroesophageal reflux disease may mean the state in which, after the subject diagnosed with gastroesophageal reflux disease have been treated through drug treatment, etc., erosion is observed in the subject by performing upper gastrointestinal endoscopy for the subject. Particularly, the recurrence of gastroesophageal reflux disease may be the state corresponding to erosive gastroesophageal reflux disease in grades A to D based on the LA grade by performing upper gastrointestinal endoscopy for the subject.

In the present invention, the prevention of the recurrence may mean that after the subject diagnosed with gastroesophageal reflux disease have been treated through drug treatment, etc., preventing, delaying or inhibiting the subject from being diagnosed with the onset of gastroesophageal reflux disease again or occurred symptoms of gastroesophageal reflux disease again.

In embodiments of the present invention, the prevention of the recurrence of gastroesophageal reflux disease may mean that after gastroesophageal reflux disease has been treated, a state in which symptoms of gastroesophageal reflux disease such as heartburn or gastric acid reflux does not exhibit again is maintained for a certain period of time.

In embodiments of the present invention, the prevention of the recurrence of gastroesophageal reflux disease may mean that after gastroesophageal reflux disease has been treated, a state in which erosion is not observed through endoscopy is maintained for a certain period of time, and particularly may mean that the state in which does not correspond to erosive gastroesophageal reflux disease in grades A to D based on LA grade through upper gastrointestinal endoscopy is maintained for a certain period of time.

The composition of the present invention may effectively prevent and/or inhibit the recurrence of gastroesophageal reflux disease even in the subject who has been diagnosed with moderate to severe gastroesophageal reflux disease. Particularly, the composition of the present invention may effectively prevent and/or inhibit the recurrence of gastroesophageal reflux disease even in the subject who has had moderate to severe symptoms of gastroesophageal reflux disease and/or the subject who has experienced erosive gastroesophageal reflux disease corresponding to C or D of LA grade.

The composition of the present invention may be administered to the subject who has been diagnosed with gastroesophageal reflux disease more than or equal to once. Particularly, the subject may be one who has been diagnosed with gastroesophageal reflux disease more than or equal to once, more than or equal to twice or more than or equal to three times, wherein the subject who has been diagnosed with gastroesophageal reflux disease more than or equal to twice might have been diagnosed with non-erosive gastroesophageal reflux disease only, erosive gastroesophageal reflux disease only, or both of non-erosive gastroesophageal reflux disease and erosive gastroesophageal reflux disease. For example, the pharmaceutical composition of the present invention may effectively prevent and/or inhibit the recurrence of non-erosive gastroesophageal reflux disease and/or erosive gastroesophageal reflux disease even in the subject who has been diagnosed with non-erosive gastroesophageal reflux disease and/or erosive gastroesophageal reflux disease more than or equal to once, more than or equal to twice or more than or equal to three times.

In embodiments of the present invention, the subject to which the pharmaceutical composition comprising 25 mg of tegoprazan is administered may be one whose gastroesophageal reflux disease is identified as having been treated after the onset of it, wherein the subject may be one who is diagnosed with gastroesophageal reflux disease within about 12 weeks before administration of the pharmaceutical composition comprising 25 mg of tegoprazan.

In embodiments of the present invention, the subject to which the pharmaceutical composition comprising 25 mg of tegoprazan is administered may be one whose gastroesophageal reflux disease is identified as having been treated after the onset of it, wherein the subject may be one who is experienced with symptoms of gastroesophageal reflux disease such as heartburn or gastric acid reflux within about 12 weeks before administration of the pharmaceutical composition comprising 25 mg of tegoprazan.

In embodiments of the present invention, the subject to which the pharmaceutical composition comprising 25 mg of tegoprazan is administered may be one whose non-erosive gastroesophageal reflux disease is identified as having been treated after the onset of it, wherein the subject may be one who has experienced symptoms of gastroesophageal reflux disease such as heartburn or gastric acid reflux, though erosion is not observed by performing upper gastrointestinal endoscopy within about 12 weeks before administration of the pharmaceutical composition comprising 25 mg of tegoprazan.

In embodiments of the present invention, the subject to which the pharmaceutical composition comprising 25 mg of tegoprazan is administered may be one whose erosive gastroesophageal reflux disease is identified as having been treated after the onset of it, wherein the subject may be one who is diagnosed with erosive gastroesophageal reflux disease, within about 12 weeks before administration of the pharmaceutical composition comprising 25 mg of tegoprazan. Particularly, the subject may be one who is diagnosed with erosive gastroesophageal reflux disease corresponding to A to D of LA grade through upper gastrointestinal endoscopy within about 12 weeks before administration of the pharmaceutical composition comprising 25 mg of tegoprazan.

In embodiments of the present invention, the subject to which the pharmaceutical composition comprising 25 mg of tegoprazan is administered may be one whose gastroesophageal reflux disease is identified as having been treated after the onset of it, wherein the subject may be one whose gastroesophageal reflux disease is identified as having been treated within about 14 days, particularly within 7 days, before administration of the pharmaceutical composition comprising 25 mg of tegoprazan.

In embodiments of the present invention, the subject to which the pharmaceutical composition comprising 25 mg of tegoprazan is administered may be one whose gastroesophageal reflux disease is identified as having been treated after the onset of it, wherein the subject may be one who has no experience in a symptom of heartburn or gastric acid reflux resulting from gastroesophageal reflux disease within about 14 days, particularly within 7 days, before administration of the pharmaceutical composition comprising 25 mg of tegoprazan.

In embodiments of the present invention, the subject to which the pharmaceutical composition comprising 25 mg of tegoprazan is administered may be one whose non-erosive gastroesophageal reflux disease is identified as having been treated after the onset of it, wherein the subject may be one who has not experienced a symptom of heartburn or gastric acid reflux resulting from gastroesophageal reflux disease within about 14 days, particularly within 7 days, before administration of the pharmaceutical composition comprising 25 mg of tegoprazan.

In embodiments of the present invention, the subject to which the pharmaceutical composition comprising 25 mg of tegoprazan is administered may be one whose erosive gastroesophageal reflux disease is identified as having been treated after the onset of it, wherein the subject may be one whose erosive gastroesophageal reflux disease is identified as having been treated within about 14 days, particularly within 7 days, before administration of the pharmaceutical composition comprising 25 mg of tegoprazan. Particularly, the subject may be one who is not identified as having erosive gastroesophageal reflux disease corresponding to A to D of LA grade through upper gastrointestinal endoscopy within about 14 days, particularly about within 7 days, before administration of the pharmaceutical composition comprising 25 mg of tegoprazan.

In embodiments of the present invention, the subject to which the pharmaceutical composition comprising 25 mg of tegoprazan is administered may maintain a state in which the onset of gastroesophageal reflux disease or a symptom of it such as heartburn or gastric acid reflux does not exhibit again for 52 weeks or more, 24 weeks or more, particularly for 4, 12, 24, 52 weeks or more, from the administration of the pharmaceutical composition.

In embodiments of the present invention, the subject to which the pharmaceutical composition comprising 25 mg of tegoprazan is administered may maintain a state in which erosive gastroesophageal reflux disease does not recur for 52 weeks or more, 24 weeks or more, particularly for 4, 12,24 and 52 weeks or more. Particularly, the subject to which the pharmaceutical composition comprising 25 mg of tegoprazan is administered may be one whose erosive gastroesophageal reflux disease corresponding to A to D of LA grade through upper gastrointestinal endoscopy for the subject is not identified for 12 to 24 weeks or more, particularly for 12, 24 and 52 weeks or more, from administration of the pharmaceutical composition.

The present invention provides the pharmaceutical composition comprising 25 mg of tegoprazan for preventing the recurrence of non-erosive gastroesophageal reflux disease by administering it to the subject whose non-erosive gastroesophageal reflux disease has been treated after being diagnosed with the non-erosive gastroesophageal reflux disease. The present invention provides a pharmaceutical composition comprising 25 mg of tegoprazan, for preventing the recurrence of erosive gastroesophageal reflux disease by administering to the subject whose erosive gastroesophageal reflux disease have been treated after being diagnosed with the erosive gastroesophageal reflux disease.

In embodiments of the present invention, the subject may be one whose gastroesophageal reflux disease has been treated by administering a drug such as a gastric-acid secretion inhibitor before the administration of the pharmaceutical composition comprising 25 mg of tegoprazan.

In embodiments of the present invention, the subject may be one who is identified as having been cured of erosive gastroesophageal reflux disease by administering a drug after the diagnosis of the erosive gastroesophageal reflux disease before administration of the pharmaceutical composition comprising 25 mg of tegoprazan.

In embodiments of the present invention, the subject may be one who is identified as having been cured of non-erosive gastroesophageal reflux disease by administering a drug after the diagnosis of the non-erosive gastroesophageal reflux disease before administration of the pharmaceutical composition comprising 25 mg of tegoprazan.

In embodiments of the present invention, the gastric-acid secretion inhibitor may be at least one of proton pump inhibitors (PPIs), antigastrin agents, anticholinergic agents and H2-blockers; particularly, at least one of cimetidine, ranitidine, famotidine, nizatidine, omeprazole, lansoprazole, esomeprazole, rabeprazole and pantoprazole; more particularly the proton pump inhibitors; and much more particularly at least one of omeprazole, lansoprazole, esomeprazole, rabeprazole and pantoprazole.

In embodiments of the present invention, the subject to which the pharmaceutical composition comprising 25 mg of tegoprazan is administered may be one who is infected with H. pylori.

In embodiments of the present invention, the pharmaceutical composition comprising 25 mg of tegoprazan may effectively prevent the recurrence of gastroesophageal reflux disease without much influence of the presence of infection with H. pylori of the subject to be administered.

In embodiments of the present invention, the subject to which the pharmaceutical composition comprising 25 mg of tegoprazan is administered may be one who has polymorphism of CYP genes. Particularly, the subject to which the pharmaceutical composition comprising 25 mg of tegoprazan is administered may be one who has at least one polymorphism of gene selected from the group consisting of CYP1A2, CYP12C9, CYP12C19, CYP12D6, CYP12E1 and CYP13A4, and more particularly may be one who has polymorphism of CYP2C19 gene and/or CYP3A4 gene. In embodiments of the present invention, the pharmaceutical composition comprising 25 mg of tegoprazan may effectively prevent the recurrence of gastroesophageal reflux disease without much influence of polymorphism of CYP gene for the subject to be administered. Particularly, the pharmaceutical composition comprising 25 mg of tegoprazan may effectively prevent the recurrence of gastroesophageal reflux disease without much influence of at least one polymorphism of gene selected from the group consisting of CYP1A2, CYP12C9, CYP12C19, CYP12D6, CYP12E1 and CYP13A4 of the subject to be administered. More particularly, the pharmaceutical composition comprising 25 mg of tegoprazan may effectively prevent the subject the recurrence of gastroesophageal reflux disease without much influence of polymorphism of CYP2C19 gene and/or CYP3A4 gene of the subject to be administered.

In embodiments of the present invention, the pharmaceutical composition comprising 25 mg of tegoprazan may be administered once to three times a day for 52 weeks or more, particularly once to three times a day for 4 to 52 weeks, more particularly once a day for 4 to 12 weeks or 4 to 24 weeks, or 4 to 52 weeks.

In embodiments of the present invention, the pharmaceutical composition comprising 25 mg of tegoprazan may be formulated into a unit dosage form. If the pharmaceutical composition comprising 25 mg of tegoprazan is formulated into a unit dosage form such as tablet, capsule or the like, the unit dosage form may be administered once to three times a day, particularly once a day regardless of meals, and may effectively prevent the recurrence of gastroesophageal reflux disease, even if it is administered at any time of the day.

In embodiments of the present invention, the pharmaceutically acceptable salts mean the salts formed with any inorganic acid, organic acid or base, which neither causes a serious stimulus to the subject to be administered nor does damage to biological activity and physical property of the compound. As the salts, salts commonly used in the art may be used, such as acid addition salts formed by the pharmaceutically acceptable free acid. The pharmaceutically acceptable salts may be selected particularly from the group consisting of pidolate salt, acetate salt, adipate salt, aspartate salt, benzoate salt, besylate salt, bicarbonate salt/carbonate salt, bisulfate salt/sulfate salt, borate salt, camsylate salt, citrate salt, cyclamate salt, edisylate salt, esylate salt, formate salt, fumarate salt, gluceptate salt, gluconate salt, glucuronate salt, hexafluorophosphate salt, hibenzate salt, hydrochloride salt/chloride salt, hydrobromide salt/bromide salt, hydroiodide salt/iodide salt, isethionate salt, lactate salt, malate salt, maleate salt, malonate salt, mesylate salt, methylsulphate salt, naphthylate salt, 2-napsylate salt, nicotinate salt, nitrate salt, orotate salt, palmitate salt, pamoate salt, phosphate salt/hydrogen phosphate salt/dihydrogen phosphate salt, pyroglutamate salt, saccharate salt, stearate salt, succinate salt, tannate salt, tartrate salt, tosylate salt, trifluoroacetate salt and xinofoate salt, but are not limited thereto. Any salts may be used without limitation, as long as they may conventionally show the pharmacological activity of the tegoprazan.

The pharmaceutical composition of the present invention for preventing the recurrence of gastroesophageal reflux disease comprising tegoprazan or a pharmaceutically acceptable salt thereof in amount of 25mg as tegoprazan may further comprise pharmaceutically acceptable additives, conventionally used appropriate carriers, excipients, disintegrants, binders, glidants or diluents.

Herein, the term "pharmaceutically acceptable additives" may include carriers, excipients, disintegrants, binders, glidants or diluents, which neither irritate organisms nor inhibit the biological activity and property of an injected compound. Types of the additives usable in the present invention are not specifically limited, and any additives may be used, as long as they are conventionally used and pharmaceutically acceptable in the art. A non-limiting example of the additives may include mannitol, microcrystalline cellulose, croscarmellose sodium, hydroxypropyl cellulose, colloidal silicon dioxide, microcrystalline cellulose, croscarmellose sodium, hydroxypropyl cellulose, colloidal silicon dioxide, magnesium stearate or mixtures thereof. In addition, other conventional additives such as antioxidants, buffers and/or bacteriostatic agents and the like may be added and used, if necessary.

The pharmaceutical composition the present invention for preventing the recurrence of gastroesophageal reflux disease comprising tegoprazan or a pharmaceutically acceptable salt thereof in amount of 25mg as tegoprazan may be formulated for oral administration, and particularly may be formulated as tablets, capsules or the like.

The present invention provides a pharmaceutical composition comprising tegoprazan or a pharmaceutically acceptable salt thereof in amount of 25mg as tegoprazan, for use in a method of preventing the recurrence of gastroesophageal reflux disease.

The present invention provides the pharmaceutical composition comprising tegoprazan or a pharmaceutically acceptable salt thereof in amount of 25mg as tegoprazan, for use in a method of preventing the recurrence of gastroesophageal reflux disease wherein the pharmaceutical composition is administered to a subject whose gastroesophageal reflux disease has been treated after being diagnosed with gastroesophageal reflux disease.

The present invention provides the pharmaceutical composition comprising tegoprazan or a pharmaceutically acceptable salt thereof in amount of 25mg as tegoprazan, for use in a method of preventing the recurrence of erosive gastroesophageal reflux disease wherein the pharmaceutical composition is administered to a subject whose erosive gastroesophageal reflux disease has been treated after being diagnosed with erosive gastroesophageal reflux disease.

Matters mentioned in the pharmaceutical composition of the present invention are also equally applied to method of treatment and uses, as long as they do not contradict each other.

### [Advantageous Effects]

The present invention may effectively prevent the recurrence of gastroesophageal reflux disease for a long period of time without any side effects by administering a pharmaceutical composition comprising tegoprazan or a pharmaceutically acceptable salt thereof in amount of 25mg as tegoprazan to a subject.

### [Description of Drawings]

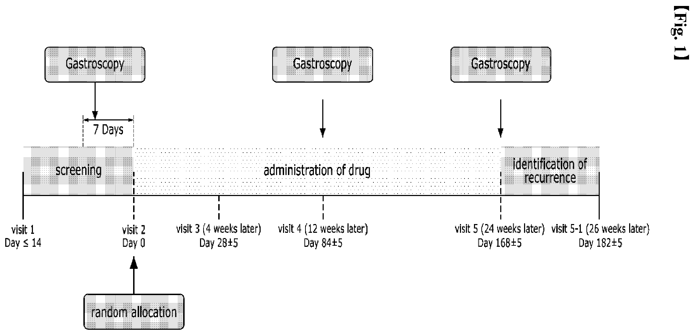

Fig. 1 schematically illustrates a clinical trial process performed using the pharmaceutical composition of the present invention.

### [Mode for Invention]

Hereinafter, the present invention will be described in more detail through embodiments. These embodiments are provided only for the purpose of illustrating the present invention in more detail.

### Preparation Example 1: Preparation of pharmaceutical formulation (1) - Tegoprazan 25 mg tablet

A formulation was prepared to contain 25 mg of 4-[(5,7-difluoro-3,4-dihydro-2H-chromene-4-yl)oxy]-N,N,2-trimethyl-1H-benzimidazole-6-carboxamide (tegoprazan) as a main component. The main component was mixed with mannitol, microcrystalline cellulose, and croscarmellose sodium; fillers were contained therein at a ratio of 1 to 99 wt% (25 mg of mannitol and 40 mg of microcrystalline cellulose) with respect to parts by weight of a final formulation; and disintegrants were prepared by using within a ratio range of 1 to 20 wt% (5 mg of croscarmellose sodium) with respect to parts by weight of the final formulation.

The mixture was granulated by adding a binder solution comprising hydroxypropyl cellulose and purified water, and content of binders was used within a range of 4 to 40 wt% (4 mg of hydroxypropyl cellulose) with respect to parts by weight of an active ingredient.

The granules were sized after the drying process, the resulting granules, microcrystalline cellulose, croscarmellose sodium, colloidal silicon dioxide and magnesium stearate were added to the granules and mixed together.

A ratio of diluents was used within a range of 1 to 10 wt% (1 mg of colloidal silicon dioxide) with respect to parts by weight of a final formulation, and a ratio of lubricant was used within a range of 1 to 10 wt% (1 mg of magnesium stearate) with respect to parts by weight of the final formulation, after which the resulting mixture was compressed to prepare a tablet.

The tablet was coated with a film coating agent. The coating was prepared to consist of a weight ratio of 2 to 6% (3 mg) with respect to parts by weight of the final formulation.

### Preparation Example 2: placebo for tegoprazan 25 mg

A placebo for tegoprazan 25 mg was prepared by means of the same method as shown in Preparation Example 1, except for not using the main component, tegoprazan.

### Preparation Example 3: Preparation of lansoprazole 15 mg formulation

In case of a lansoprazole formulaton, Lanston cap. 15 mg (lansoprazole 15 mg) of Jeil Pharmaceutical Co., Ltd. was purchased and used.

### Preparation Example 4: Placebo for Lansoprazole 15 mg

A placebo for lansoprazole 15 mg as prepared by means of the same method as a method for preparing a Lanston cap. by adding pharmaceutically acceptable excipients thereto, except for not using the main component, lansoprazole.

### Preparation Example 5:

In case of an esomeprazole formulation, Nexium tab. 20 mg (esomeprazole 20 mg) of Astrazeneca Korea Co., Ltd. was purchased and used.

### Example 1

### 1. Usage, dosage, administration period and selection of subjects

Tegoprazan and esomeprazole were orally administered for 7 days as shown in table 1 (an open-label, active controlled, parallel design and repeated dose).

**[Table 1]**

| Usage, dosage and number of subjects | | |
|---|---|---|
| Group | Usage and Dosage | Number of trial subjects |
| Group 1 | To administer 25 mg of tegoprazan once a day for seven days | 8 |
| Group 2 | To administer esomeprazole 20 mg once a day for seven days | 8 |

### Inclusion criteria

Trial subjects should satisfy all the following selection criteria in order to participate in this clinical trial, unless otherwise specified:
(1) a healthy male volunteers in the age between 19 and 50 years old (inclusive);
(2) a person who had a body mass index (BMI) between 18 kg/m² and 27 kg/m² (inclusive) and weighting between 55 kg and 90 kg (inclusive) at the time of screening test;
(3) a person who is negative on Helicobacter pylori;
(4) a person who agrees to use effective contraceptive methods and to not provide sperm, from the first administration of a clinical trial drug to 30 days after the last administration of a clinical trial drug; and
(5) a person decided to be inappropriate to participate in this trial according to investigator on the result of physical examination, clinical laboratory test, medical examination by interview, etc.

### Exclusion criteria

The trial subjects were excluded from this clinical trial, in the event that any of the followings occurs:
(1) a person with the medical history of clinically significant hepatic, renal, neurologic, respiratory, endocrine, haematologic, oncologic, cardiovascular, urinary and psychiatric diseases;
(2) a person with the medical history of gastrointestinal diseases (gastrointestinal ulcer, gastritis, gastrospasm, gastroesophageal reflux disease, Crohn's disease, etc.) or surgery (excluding uncomplicated appendectomy or herniotomy) affecting the safety and pharmacodynamics of a clinical trial drug;
(3) a person with hypersensitivity to drugs containing proton pump inhibitor-based components and other drugs (aspirin, antibiotics, etc) or history of clinically significant hypersensitivity;
(4) a person with a positive reaction to a serum test (hepatitis B, human immunodeficiency virus (HIV) and hepatitis C test);
(5) a person with total bilirubin, AST (GOT) and ALT (GPT) in the blood more than 1.5 times of the reference upper limit in a previous screening test including an additional test before random allocation;
(6) a person who had systolic blood pressure of less than 90 mmHg or more than 140 mmHg; diastolic blood pressure of less than 50 mmHg or more than 95 mmHg; and pulse rate of less than 45 beats/min or more than 100 beats/min, according to vital signs measured in the sitting position after the break for at least five minutes in a previous screening test prior to random allocation;
(7) a person who had anatomical abnormalities for insertion and maintenance of a pH meter catheter, or expected not to withstand the insertion of the pH meter catheter;
(8) a person with the medical history of drug abuse or positive reaction to abused drugs in a urine screening test;
(9) a person who has taken any prescription drugs (ETCs), or drugs (including proton pump inhibitors, H2-receptor antagonists, antacids, etc.) or herbal medicines affecting a gastric pH within two weeks before the expected first dose; or use of any over-the-counter drugs (OTC), health functional food or vitamins within one week therebefore (however, eligible to participate in the clinical trial, in case of satisfying other conditions depending on the investigator's decision), or expected to do so;
(10) a person who had enrolled to participate in different bioequivalence studies or other clinical trials within three months before the expected first dose;
(11) a person who had donated blood within two months before the expected first dose or who donated substance of blood within one month therebefore, or blood transfusion within one month therebefore;
(12) a person who takes Excessive caffeine (more than 5 units/day) or persistent alcohol (more than 21 units/week, 1 unit = 10 g of pure alcohol), or unable to prevent drinking during a hospitalization period;
(13) a person with cotinine positive in a urine screening test or who can't stop smoking during a whole period of the clinical trials;
(14) a person who has taken the food containing grapefruit during a period from 24 hours before hospitalization until discharging from the hospital, and can't stop taking the food containing grapefruit during this period;
(15) a person who can't stop taking the food containing caffeine (coffee, tea (black tea, green tea, etc.), carbonated beverage, coffee-flavored milk, nutritional tonic drinks and the like) during a period from 24 hours before hospitalization until discharging from the hospital;
(16) a person who can't use medically acceptable methods of contraception during a whole period of the clinical trials; and
(17) a person who is determined to be unsuitable about participation in the clinical trials by the investigator for other reasons as a result of the clinical laboratory test.

### 2. Evaluation

There occurred neither serious adverse reactions nor dropouts caused thereby while this clinical trial was in progress. With regard to a gastric pH, a time for which the gastric pH exceeds 4 was evaluated during an observation period for the gastric pH.

After evaluating pharmacokinetic parameters, the time (%) for which a pH of gastric fluid exceeds and maintains 4 based on 24 hours is the same as shown in the following table 2.

**[Table 2]**

| Evaluation values of pharmacokinetic parameters upon administering the formulation of Example 1 (25 mg of tegoprazan) | | |
|---|---|---|
| HTR > pH4 (%) | 25 mg of tegoprazan | |
| | Day 1 | Day 7 |
| Mean | 40.6% | 56.6% |
| SD | 7.6% | 17.9% |

As identified in the table above, a change in pH was observed for 24 hours upon administering 25 mg of tegoprazan. During an observation period for a gastric pH, it was shown in a group dosed with 25 mg of tegoprazan that an average of time for which the gastric pH exceeds 4 (Time pH > 4, %) was at least 40% from day 1 after the administration, and thus it was clearly observed that gastric acidity is inhibited due to the administration of 25 mg of tegoprazan.

### Example 2

### 1. Selection of subjects

A double-blinded, randomized and active controlled study was designed to identify an effect of 25 mg of tegoprazan on maintenance therapy for gastroesophageal reflux disease.

### Inclusion criteria

Trial subjects should satisfy all the following selection criteria in order to participate in this clinical trial, unless otherwise specified:
(1) a healthy volunteer in the age between 20 and 75 years old (inclusive);
(2) a person who is diagnosed with erosive gastroesophageal reflux disease (LA grade A-D) through upper gastrointestinal endoscopy within 12 weeks based on random group allocation (visit 2);
(3) a person who is identified as having erosive gastroesophageal reflux disease treated through upper gastrointestinal endoscopy within seven days based on random group allocation (visit 2) (a person who is identified the cure of erosion after administration of a standard dose of therapeutic agents for erosive gastroesophageal reflux disease (PPI, P-CAB, etc.) for 4-8 weeks due to a typical symptom of gastroesophageal reflux disease and erosive gastroesophageal reflux disease);
(4) a person who is not identified as having heartburn (heartburn, burning sensation or pains inside the breastbone) and gastric acid reflux (a symptom of having acid reflux or having a content of the stomach flowing back up into the esophagus) within seven days, based on random group allocation (visit 2);
(5) a person who able to understand and follow the instructions and participate in a whole period of the clinical trial;
(6) a person who decided voluntarily to participate in the clinical trial and agreed to observe notice; and
(7) a person who agrees to use medically adequate contraceptive methods (including medically infertile states) during a period of the clinical trial.

### Exclusion criteria

The trial subjects were excluded from this clinical trial, in the event that any of the followings occurs:
(1) a person who can't receive an upper gastrointestinal endoscopy;
(2) a person who had esophageal stenosis, ulcer stenosis, esophagogastric varix, long segment Barrett esophagus (LSBE) exceeding 3 cm in length, active peptic ulcer, gastrointestinal tract bleeding or malignant tumor, upon the upper gastrointestinal endoscopy;
(3) a person who had "premonitory symptom" enough to presume a malignant disease in the gastrointestinal tract, for example, symptoms such as odynophagia, serious dysphagia, bleeding, weight loss, anemia and bloody stool (excluding piles) (however, even those showing the premonitory symptom may be included in this clinical trial, as long as the presence of a tumor is diagnosed with negative as a result of endoscopy.);
(4) a person who is diagnosed with eosinophilic oesophagitis - however, those determined as having no eosinophilic oesophagitis through esophagus biopsy may be included in this clinical trial;
(5) a person who is diagnosed with primary esophageal motility disorder, irritable bowel syndrome (IBS), inflammatory bowel disease (IBD), etc. within the recent three months or suspected of having IBS (excluding the patients suspected of having IBS after the investigator's medical examination about abdominal discomfort, stool viscosity, number of bowel movement, etc.);
(6) a person who is experienced in undergoing surgery for inhibition of gastric acid secretion or a gastroesophageal surgery in the past (excluding simple perforation surgery, appendectomy, cholecystotomy, and resection of a benign tumor using an endoscope);
(7) a person who had acquired immune deficiency syndrome (AIDS) or hepatitis (including hepatitis virus carriers: HBs-antigen positive or HCV-antibody positive) (HCV-antigen or HCV-RNA negative subjects are eligible to participate in this clinical trial);
(8) a person who had anamnesis of mental diseases within the recent five years;
(9) a person who is required to persistently take nonsteroidal anti-inflammatory drugs (NTHEs), antithrombotics, etc. during the clinical trial period (however, the patients are allowed to take a low dose of aspirin (100 mg or less per day, if they have taken it from before participating in this clinical trial just for the purpose of prevention.);
(10) a person who are administering HIV protease inhibitors (Atazanavir and Nelfinavir) or Rilpivirine-comprising preparations;
(11) Pregnant or breast-feeding;
(12) a person who has shown clinically significant abnormal values in a screening test;
   - Values of AST, ALT, ALP, γ-GT and total bilirubin equal to or more than double the upper normal limit (UNL) for each testing institution;
   - Values of BUN and creatinine equal to or more than 1.5 times the UNL for each testing institution;
(13) a person who has shown clinically significant abnormal electrocardiogram (ECG) (major arrhythmia, multifocal PVC, 2° AV-block abnormalities, etc.);
(14) a person with Zollinger-Ellison syndrome;
(15) a person who had anamnesis of malignant tumor within the recent five years (however, this clinical trial may include those who were determined as having a complete remission from the tumor (CR, pCR) and have not had any recurrence thereof for at least five years from the day of such determination, and those who had a tumor completely removed through endoscopic excision and have not had any recurrence thereof for at least three years.);
(16) a person who has shown clinically significant disorders in hepatic, renal, cardiovascular, respiratory, endocrine, and central nervous systems;
(17) a person with hypersensitivity to components of the clinical trial drug (including expellants) or benzimidazoles and anamnesis thereof;
(18) a person who has scheduled to receive a surgery requiring hospitalization or needing a surgical treatment during participation in this clinical trial;
(19) a person how has participated in other clinical trials within the recent 12 weeks based on the start day of the screening (visit 1) (however, this clinical trial may allow the participation of clinical trial subjects, who used to participate or have been now participating in non-interventional studies (such as observational study, questionnaire study, etc.), considered not to affect the validity and safety of this clinical trial by the investigator, or clinical trial subjects, who dropped out from the screening for administration of and treatment with the clinical trial drug after filling in the consent form to participate in other clinical trials.)

### 2. Clinical trial design

The clinical trial was performed in a double-blinded, randomized, active-controlled and multi-center clinical trial manner, and the clinical trial may be schematized as shown in Fig. 1.

Subjects, who paid a visit for the clinical trial, were selected by receiving the screening numbers in the order agreed on the first day of visit (visit 1), performing screening test, checking their medical history, and performing gastroscopy according to the select criteria. The completely selected clinical trial subjects were randomly allocated (visit 2) and classified into two groups.

The subjects were classified using stratified block randomization based on LA grades identified by upper gastrointestinal endoscopy performed within 12 weeks before the random allocation on visit (visit 2). Each group was administered with the drug for 24 weeks.

### 3. Dosage and method

The clinical trial subjects received tablets and capsules as shown in table 3.

**[Table 3]**

| | |
|---|---|
| Test group | Preparation Example 1 + Preparation Example 4 |
| Control group | Preparation Example 2 + Preparation Example 3 |

As identified in the table above, the test group was orally dosed with 25 mg of tegoprazan tablet (Preparation Example 1)/lansoprazole 15 mg placebo (Preparation Example 4), and the control group was orally dosed with 25 mg of tegoprazan placebo (Preparation Example 2)/lansoprazole 15 mg capsule (Preparation Example 3).

The subjects randomly allocated to each group took the prescribed clinical trial drug once a day at a regular time from the first day of prescription. A diary on the trial subjects was kept from the start day of taking the clinical trial drug.

If the moderate to severe symptoms of heartburn or gastric acid reflux were observed for three consecutive days during an administration period of the clinical trial drug, it was checked whether erosive gastroesophageal reflux disease recurred or not through an endoscopy. If the recurrence of erosion was identified through the endoscopy, a corresponding subject was terminated in the clinical trial. However, if the symptoms were somewhat alleviated by taking expellant not PPIs, the clinical trial was continuously performed in such a way that the expellant rather than the PPIs had to be limited to maximum once a day, but not exceeding twice a week (however, the expellent were not taken within one hour before and after taking off the clinical trial drug).

During a visit in 4 weeks (visit 3), 12 weeks (visit 4) and 24 weeks (visit 5) later, subjects paid a visit on an empty stomach without taking the clinical trial drug, then received a scheduled test, then were newly prescribed the same drug, then took it from the very day, then received a test in their visit in 24 weeks (visit 5) later, and then stopped the administration.

In four weeks (visit 3) after administering the clinical trial drug, endoscopy was not performed, but clinical laboratory tests (hematologic test, blood chemistry test, blood coagulation test, urine test, hepatitis test, etc.) and evaluation of symptoms were performed. In 12 weeks (visit 4) and 24 weeks (visit 5) later, it was checked whether erosion recurred or not through the upper gastrointestinal endoscopy, and the clinical laboratory test, evaluation of symptoms and the like were performed. Clinical trial subjects took the clinical trial drug for up to 24 weeks and then entered into a safety follow-up (f/u) period, in which the f/u was performed (by phone or visit) in two weeks after the last administration.

### 4. Evaluation method

### A. Primary efficacy outcome ... Endoscopic remission maintenance rate in 24 weeks later

① Endoscopic remission: No recurrence of erosion (LA grade A-D) observed through upper gastrointestinal endoscopy during a maintenance period (24 weeks) Endoscopic remission maintenance rate in 24 weeks later (%) = (Number of clinical trial subjects without any recurrence of erosion observed during the maintenance period/Number of clinical trial subjects having received an upper gastrointestinal endoscopy during the maintenance period after administration of the clinical trial drug) × 100
   - However, if the endoscopic recurrence was identified, a corresponding clinical trial subject was terminated in the clinical trial at the time of such identification and was included in the total number of clinical trial subjects.
   - If a clinical trial subject without any recurrence of erosion observed through upper gastrointestinal endoscopy failed to paid a visit on a due day (> 168+5 days) in 24 weeks (± 5 days) after administration of the clinical trial drug, this case was included in an endoscopic remission maintenance rate in 24 weeks later.

### B. Secondary efficacy outcome ... Endoscopic remission maintenance rate in 12 weeks later

① Endoscopic remission: No recurrence of erosion (LA grade A-D) observed through upper gastrointestinal endoscopy during a maintenance period (12 weeks) Endoscopic remission maintenance rate in 12 weeks later (%) = (Number of clinical trial subjects without any recurrence of erosion observed during the maintenance period / Number of clinical trial subjects having received an upper gastrointestinal endoscopy during the maintenance period after administration of the clinical trial drug) × 100
   - However, if the endoscopic recurrence was identified, a corresponding clinical trial subject was terminated in the clinical trial at the time of such identification and was included in the total number of clinical trial subjects.
   - If a clinical trial subject without any recurrence of erosion observed through upper gastrointestinal endoscopy failed to pay a visit on a due day (> 84+5 days) after 12 weeks (± 5 days) of the administration of the clinical trial drug, this case was included in an endoscopic remission maintenance rate in 12 weeks later.

### C. Additional efficacy outcome

### 1) Rate of subjects without main symptoms (heartburn and/or gastric acid reflux) in 4, 12 and 24 weeks later

① Rate of subjects without main symptoms (heartburn and gastric acid reflux) Rate of subjects without main symptoms = (Subjects without main symptoms expressed/Number of clinical trial subjects whose symptoms were evaluated during a maintenance period after administration of the clinical trial drug) × 100
② Rate of subjects without heartburn Rate of subjects without heartburn = (Subjects without heartburn expressed/Number of clinical trial subjects whose symptoms were evaluated during a maintenance period after administration of the clinical trial drug) × 100
③ Rate of subjects without gastric acid reflux Rate of subjects without gastric acid reflux = (Subjects without gastric acid reflux expressed/Number of clinical trial subjects whose symptoms were evaluated during a maintenance period after administration of the clinical trial drug) × 100

### 2) Evaluation of symptoms through a diary of clinical trial subjects in 4, 12 and 24 weeks later

① Rate of days without main symptoms (heartburn and gastric acid reflux) Rate of days without main symptoms = (Number of days without main symptoms expressed/Number of days evaluating main symptoms) × 100
② Rate of days without heartburn Rate of days without heartburn = (Number of days without main symptoms expressed/ Number of days evaluating main symptoms) × 100
③ Rate of days without gastric acid reflux Rate of days without gastric acid reflux = (Number of days without main symptoms expressed (day + night)/Number of days evaluating main symptoms) × 100

Evaluation of symptoms in each visit and through a diary of clinical trial subjects was evaluated using the following scale.

**[Table 4]**

| | |
|---|---|
| 0 (none) | No symptom |
| 1 (mild) | A symptom, but not disrupting daily activities including sleep |
| 2 (moderate) | Slight discomfort, disrupting daily activities including sleep |
| 3 (severe) | Symptoms occurred in most of time, frequently disrupting daily activities including sleep |

### 3) GERD-HRQL evaluation

: Average amount of change in GERD-HRQL (Gastro-Esophageal Reflux Disease Health Related Quality Life) total scores in 4, 12 and 24 weeks later compared to baseline before administration

Subjects were asked to fill in the questionnaires on every visit according to Velanovich V. (The development of the GERD-HRQL symptom severity instrument. DisEsophagus 2007;20:130-4), and the scores of GERD-HRQL are defined as follows.

**[Table 5]**

| | |
|---|---|
| 0 | No symptom |
| 1 | A symptom, but not disrupting daily activities including sleep |
| 2 | Slight discomfort, disrupting daily activities including sleep |
| 3 | Symptoms occurred in most of the time, frequently disrupting daily activities including sleep |
| 4 | Symptoms affecting daily life |
| 5 | Symptoms disrupting daily activities |

| | |
|---|---|
| Total score: 0-50 | |

### D. Subgroup analysis & evaluation

1) Endoscopic remission maintenance rate in 24 weeks later for each LA grade
2) Endoscopic remission maintenance rate in 24 weeks later for each presence of infection with H. pylori
3) Endoscopic remission maintenance rate in 24 weeks later for each use of expellants
4) Endoscopic remission maintenance rate for each CY2C19 genotype
   - Extensive Metabolisers (EM)
   - Intermediate Metaboliseres (IM)
   - Poor Metaboliser (PM)

### E. Safety evaluation

Adverse reactions, clinical laboratory tests (hematologic test, blood chemistry test, blood coagulation test and urine test), vital signs (blood pressure in sitting position, heart rate and body temperature), ECG test, and central laboratory tests (Gastrin, Pepsinogen I/II, Vitamin B12, Folate, Iron, Mg)

### 5. Evaluation results

### A. Evaluation of symptoms after 4-week administration

After both the test group and the control group were administered with a drug for four weeks, the subject administered with 25 mg of tegoprazan demonstrated a maintenance effect.

Particularly, all the subjects of the test group (administration of 25 mg of tegoprazan) and the control group (administration of 15 mg of lansoprazole) responded to the symptom evaluation after four weeks of administration of the drug that they had not experienced symptoms of heartburn and gastric acid reflux while taking the drug for four weeks.

### B. Endoscopy results after 12-week administration

The endoscopic remission maintenance rates of the test group and the control group after administering a drug for twelve weeks were evaluated (secondary efficacy outcome). A maintenance effect was observed by the subject who had been administered with 25 mg of tegoprazan.

Particularly, erosion was observed in only one subject among the total 37 subjects of the test group (administration of 25 mg of tegoprazan; at least 17 subjects) and the control group (administration of 15 mg of lansoprazole) after administering the drug for twelve weeks by upper gastrointestinal endoscopy; erosion was not observed in the remaining 36 subjects by upper gastrointestinal endoscopy.

Accordingly, it has been observed that the administration of 25 mg of tegoprazan effectively inhibits the recurrence of gastroesophageal reflux disease.

### C. Safety evaluation

No adverse reaction was observed in the subjects administered with a drug for four or twelve weeks according to the safety evaluation in terms of adverse reactions, clinical laboratory tests (hematologic test, blood chemistry test, blood coagulation test and urine test), vital signs (blood pressure in sitting position, heart rate and body temperature), ECG test, and central laboratory tests (Gastrin, Pepsinogen I/II, Vitamin B12, Folate, Iron, Mg).

## Claims

1. A pharmaceutical composition for use in a method of preventing the recurrence of gastroesophageal reflux disease, comprising tegoprazan which is a compound represented by the chemical formula 1 or a pharmaceutically acceptable salt thereof in amount of 25mg as tegoprazan:

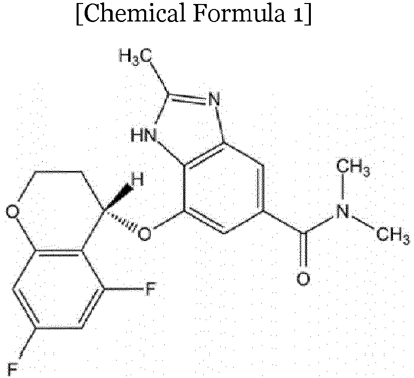

2. The pharmaceutical composition for use, according to claim 1, wherein the pharmaceutical composition is administered to a subject, whose gastroesophageal reflux disease have been treated after being diagnosed with the gastroesophageal reflux disease.

3. The pharmaceutical composition for use, according to claim 2, wherein the subject is one who has been diagnosed of gastroesophageal reflux disease more than or equal to once or twice before administration of the pharmaceutical composition.

4. The pharmaceutical composition for use, according to claim 2, wherein the subject is one, who does not show a symptom of heartburn and gastric acid reflux due to administration of a drug, after being diagnosed with gastroesophageal reflux disease before administration of the pharmaceutical composition, wherein the drug is a gastric-acid secretion inhibitor.

5. The pharmaceutical composition for use, according to claim 2, wherein the pharmaceutical composition is administered to a subject, whose erosive gastroesophageal reflux disease have been treated after being diagnosed with erosive gastroesophageal reflux disease.

6. The pharmaceutical composition for use, according to claim 5, wherein the subject is one, whose cure of erosive gastroesophageal reflux disease have been identified due to administration of a drug after being diagnosed with the erosive gastroesophageal reflux disease before administration of the pharmaceutical composition, wherein the drug is a gastric-acid secretion inhibitor.

7. The pharmaceutical composition for use, according to claim 2, wherein the pharmaceutical composition is administered to a subject whose non-erosive gastroesophageal reflux disease has been treated after being diagnosed with non-erosive gastroesophageal reflux disease.

8. The pharmaceutical composition for use, according to claim 7, wherein the subject is one whose cure of non-erosive gastroesophageal reflux disease has been identified due to administration of a drug after being diagnosed with the non-erosive gastroesophageal reflux disease before administration of the pharmaceutical composition, wherein the drug is a gastric-acid secretion inhibitor.

9. The pharmaceutical composition for use, according to claim 1, wherein the pharmaceutical composition is administered once a day for 4 to 52 weeks.

10. The pharmaceutical composition for use, according to claim 1, wherein the pharmaceutical composition is administered once a day for 4 to 24 weeks.

## Patentansprüche

1. Pharmazeutische Zusammensetzung zur Verwendung in einem Verfahren zur Vorbeugung des Wiederauftretens von gastroösophagealer Reflux-Krankheit, umfassend Tegoprazan, das eine durch die chemische Formel 1 dargestellte Verbindung ist, oder ein pharmazeutisch akzeptables Salz davon, in einer Menge von 25 mg Tegoprazan:

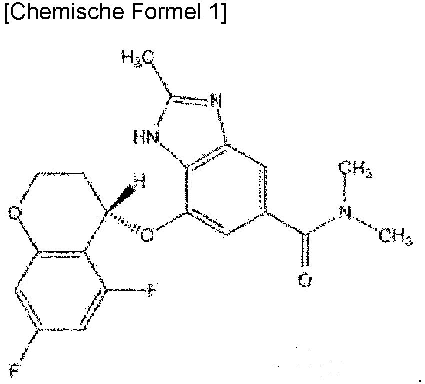

2. Pharmazeutische Zusammensetzung zur Verwendung nach Anspruch 1, wobei die pharmazeutische Zusammensetzung einem Individuum verabreicht wird, dessen gastroösophageale Reflux-Krankheit behandelt worden ist, nachdem gastroäsophageale Reflux-Krankheit diagnostiziert wurde.

3. Pharmazeutische Zusammensetzung zur Verwendung nach Anspruch 2, wobei das Individuum eines ist, bei dem gastroösophageale Reflux-Krankheit mehr als oder gleich einmal oder zweimal vor der Verabreichung der pharmazeutischen Zusammensetzung diagnostiziert worden ist.

4. Pharmazeutische Zusammensetzung zur Verwendung nach Anspruch 2, wobei das Individuum eines ist, das aufgrund der Verabreichung eines Arzneimittels kein Symptom von Sodbrennen und Magensäurerückfluss zeigt, nachdem gastroösophageale Reflux-Krankheit vor der Verabreichung der pharmazeutischen Zusammensetzung diagnostiziert wurde, wobei das Arzneimittel ein Magensäuresekretionshemmer ist.

5. Pharmazeutische Zusammensetzung zur Verwendung nach Anspruch 2, wobei die pharmazeutische Zusammensetzung einem Individuum verabreicht wird, dessen erosive gastroösophageale Reflux-Krankheit behandelt worden ist, nachdem erosive gastroösophageale Reflux-Krankheit diagnostiziert wurde.

6. Pharmazeutische Zusammensetzung zur Verwendung nach Anspruch 5, wobei das Individuum eines ist, bei dem aufgrund der Verabreichung eines Arzneimittels, nachdem erosive gastroäsophageale Reflux-Krankheit vor der Verabreichung der pharmazeutischen Zusammensetzung diagnostiziert wurde, Heilung festgestellt wurde, wobei das Arzneimittel ein Magensäuresekretionshemmer ist.

7. Pharmazeutische Zusammensetzung zur Verwendung nach Anspruch 2, wobei die pharmazeutische Zusammensetzung einem Individuum verabreicht wird, dessen nicht-erosive gastroäsophageale Reflux-Krankheit behandelt worden ist, nachdem nicht-erosive gastroösophageale Reflux-Krankheit diagnostiziert wurde.

8. Pharmazeutische Zusammensetzung zur Verwendung nach Anspruch 7, wobei das Individuum eines ist, bei dem aufgrund der Verabreichung eines Arzneimittels, nachdem nicht-erosive gastroösophageale Reflux-Krankheit vor der Verabreichung der pharmazeutischen Zusammensetzung diagnostiziert wurde, Heilung festgestellt wurde, wobei das Arzneimittel ein Magensäuresekretionshemmer ist.

9. Pharmazeutische Zusammensetzung zur Verwendung nach Anspruch 1, wobei die pharmazeutische Zusammensetzung einmal am Tag für 4 bis 52 Wochen verabreicht wird.

10. Pharmazeutische Zusammensetzung zur Verwendung, nach Anspruch 1, wobei die pharmazeutische Zusammensetzung einmal am Tag für 4 bis 24 Wochen verabreicht wird.

## Revendications

1. Composition pharmaceutique destinée à être utilisée dans un procédé de prévention de la récidive du reflux gastro-œsophagien, comprenant du tégoprazan qui est un composé représenté par la formule chimique 1 ou un sel pharmaceutiquement acceptable de celui-ci en quantité de 25 mg en tant que tégoprazan :

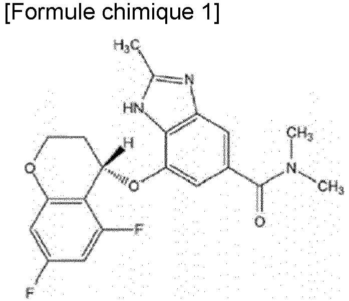

2. Composition pharmaceutique selon la revendication 1, dans laquelle la composition pharmaceutique est administrée à un sujet dont le reflux gastro-œsophagien a été traité après avoir été diagnostiqué avec le reflux gastro-œsophagien.

3. Composition pharmaceutique selon la revendication 2, dans laquelle le sujet a reçu un diagnostic de reflux gastro-œsophagien plus ou égal à une ou deux fois avant l'administration de la composition pharmaceutique.

4. Composition pharmaceutique selon la revendication 2, dans laquelle le sujet ne présente pas de symptômes de brûlures d'estomac et de reflux d'acide gastrique dus à l'administration d'un médicament, après avoir été diagnostiqué avec une maladie de reflux gastro-œsophagien avant l'administration de la composition pharmaceutique, dans laquelle le médicament est un inhibiteur de sécrétion d'acide gastrique.

5. Composition pharmaceutique selon la revendication 2, dans laquelle la composition pharmaceutique est administrée à un sujet dont le reflux gastro-œsophagien érosif a été traité après avoir été diagnostiqué avec un reflux gastro-œsophagien érosif.

6. Composition pharmaceutique selon la revendication 5, dans laquelle le sujet est celui dont la guérison du reflux gastro-œsophagien érosif a été identifiée grâce à l'administration d'un médicament après avoir été diagnostiqué avec le reflux gastro-œsophagien érosif avant l'administration de la composition pharmaceutique, dans laquelle le médicament est un inhibiteur de sécrétion d'acide gastrique.

7. Composition pharmaceutique selon la revendication 2, dans laquelle la composition pharmaceutique est administrée à un sujet dont le reflux gastro-œsophagien non érosif a été traité après le diagnostic de reflux gastro-œsophagien non érosif.

8. Composition pharmaceutique selon la revendication 7, dans laquelle le sujet est un sujet dont la guérison du reflux gastro-œsophagien non érosif a été identifiée grâce à l'administration d'un médicament après avoir été diagnostiqué avec le reflux gastro-œsophagien non érosif avant l'administration de la composition pharmaceutique, dans laquelle le médicament est un inhibiteur de sécrétion d'acide gastrique.

9. Composition pharmaceutique selon la revendication 1, dans laquelle la composition pharmaceutique est administrée une fois par jour pendant 4 à 52 semaines.

10. Composition pharmaceutique selon la revendication 1, dans laquelle la composition pharmaceutique est administrée une fois par jour pendant 4 à 24 semaines.
